# EUROPEAN PATENT APPLICATION

(11) **EP 4 296 277 A1**
(43) Date of publication of application: **27.12.2023**
(21) Application number: 22749282.4
(22) Date of filing: 23.03.2022
(51) Int. Cl.: C07K 14/435, C07K 1/34

(54) **METHOD FOR USING TANGENTIAL FLOW ULTRAFILTRATION TECHNOLOGY TO PREPARE CONTROLLABLE HIGH CONCENTRATION SILK FIBROIN SOLUTION**

(30) Priority: 08.02.2021 CN 202110171751
(71) Applicant: Favorsun Medical Tech. (Suzhou) co., Ltd., Jiangsu 215000 (CN)
(72) Inventor: YANG, Wenhua, Suzhou, Jiangsu 215000 (CN); OUYANG, Cong, Suzhou, Jiangsu 215000 (CN); LIU, Yezhuo, Suzhou, Jiangsu 215000 (CN)
(74) Representative: Gille Hrabal Partnerschaftsgesellschaft mbB Patentanwälte
(86) International application number: PCT/CN2022/082618
(87) International publication number: WO 2022/167003

(57) **Abstract**

The present invention proposes a method for preparing a controllable high-concentration silk fibroin solution with high molecular weight using tangential flow ultrafiltration technology. The method comprises: acquiring an inorganic salt solution of silk fibroin; subjecting the inorganic salt solution of silk fibroin to membrane clarification process; desalting the inorganic salt solution of silk fibroin by filtering the clarified solution obtained from the membrane clarification process through a tangential flow filtration system; and concentrating the silk fibroin solution to give a high-concentration aqueous solution of silk fibroin with high molecular weight. Compared with the prior art, the present invention achieves the desalination and concentration of a salt solution of silk fibroin by the tangential flow filtration technology, and directly acquires an aqueous solution of silk fibroin with a number-average molecular weight of 80 kDa-200 kDa. The obtained material has improved properties in all aspects. The method of the present invention greatly improves the efficiency and reduces the cost of producing silk fibroin solutions, and further more provides more convenience for the development of silk fibroin materials.

## Description

Th present application claims priority to the prior application of the Patent Application for Invention with the application No. 202110171751.X and entitled "METHOD FOR PREPARING CONTROLLABLE HIGH-CONCENTRATION SILK FIBROIN SOLUTION BY TANGENTIAL FLOW ULTRAFILTRATION TECHNOLOGY" filed with the China National Intellectual Property Administration on February 8, 2021, which is incorporated herein by reference in its entirety.

### TECHNICAL FIELD

The present invention relates to the technical field of silk fibroin processing, and particularly to a method for preparing a controllable high-concentration silk fibroin solution by tangential flow ultrafiltration technology.

### BACKGROUND

Silk is one of the strongest natural fibers and consists of 70%-80% silk fibroin and 20%-30% sericin. In addition to being used as a conventional textile material, silk can also be used as an advanced biopolymer material. Since sericin can induce inflammatory reactions in the body, it needs to be removed by processes such as degumming before silk can be used as a biomaterial. Extensive research has shown that the silk fibroin obtained from treating silk with processes such as degumming has good biocompatibility, with *in vivo* inflammatory reactions being much less intense than that of commonly used biomaterials such as collagen and polylactic acid. As a natural material with excellent physical and chemical properties, silk fibroin has excellent biocompatibility and does not cause biological rejection reactions whether attached to the body surface or implanted in the body. Meanwhile, as a natural protein, silk fibroin products can be controllably degraded within a preset time and fully absorbed by the human body after implantation. Thus, silk fibroin, as a natural macromolecular protein, has broad prospects for applications in disciplines such as physics, electronics, optics, biology, and engineering.

In recent years, with increasing attention from scientists and researchers, silk fibroin materials in the laboratory are also increasingly being used in a wide range of applications. Silk fibroin materials have been applied interdisciplinarily across fields such as material science, optoelectronic informatics, engineering, medicine and pharmaceuticals, and advanced micro-electro-mechanical system processing technology. These applications have led to the development of a series of high-tech products such as integrated sensors and biomedical devices involved in human activities, e.g., silk fibroin bone nails, artificial dura maters, sustained-release micro-needles, coronary artery stents, artificial bones, and other high-end medical devices. Silk fibroin products have broad applications in implantable medical devices such as orthopedics, sports medicine, cardiology, neurosurgery, and plastic surgery; and the potential market size for these applications is enormous. However, considering various literature and research outcomes, these achievements are mostly limited to the laboratory stage and cannot be sustained on scales beyond scientific research due to high production costs. Therefore, there is an urgent need to scale up the production of these products from the laboratory and to produce silk fibroin raw materials on a large scale.

Protein process technology is a technique for concentrating biomacromolecules, which aims to significantly increase the protein concentration per unit volume in a solution by removing water, ions, and other small molecular substances using physical or chemical methods. Many protein samples undergo a series of separation and extraction steps, leading to sample dilution and the introduction of a large number of ions. However, many analyses and studies require samples with high purity or high protein concentration, making it crucial to select an appropriate desalination and concentration method. Common desalination and concentration methods for macromolecular proteins include dialysis, electrodialysis, and ultrafiltration. Dialysis and electrodialysis methods are time-consuming, require significant sample dilution, and are not suitable for large-scale production, resulting in limited industrial application. A common method for preparing an aqueous regenerated silk fibroin solution described in China Patent CN102167724B filed by the team of the inventor of the present application is as follows: dissolving silk or waste silk in a specific solvent (including an aqueous lithium bromide solution, an aqueous lithium thiocyanate solution, an aqueous sodium thiocyanate solution, a calcium chloride-ethanol-water mixed solvent, a calcium nitrate-methanol-water mixed solvent, a lithium bromide-ethanol-water mixed solvent, ect.); and dialyzing a silk fibroin solvent in purified water using a dialysis membrane or dialysis bag to remove small solvent molecules to obtain an aqueous silk fibroin solution. Due to the high osmotic pressure caused by a considerable concentration gradient existing on both sides of a semipermeable membrane in the dialysis process, a large amount of water passes through the semi-permeable membrane into the silk fibroin solution and decreases the concentration of the silk fibroin, which may even lead to the rupture of the dialysis bag (membrane) in severe cases. The concentration of the regenerated silk fibroin solution obtained by this method generally does not exceed 5 wt%, and a low-concentration regenerated silk fibroin solution is severely limited both in the operability of manual spinning, and in its storage and transportation as a raw material for production.

China Patent CN102167724B proposes a new solution: dialyzing silk fibroin dissolved in an inorganic salt solution with an aqueous solution of a water-soluble polymer, wherein the polymer has a molecular weight of 6000-100,000 and has a concentration of 5%-50%. In this method, the water-soluble polymer may be polyethylene glycol (PEG) or polyethylene oxide (PEO), polyvinyl alcohol (PVA), polyvinyl pyrrolidone (PVP), etc. However, in practical applications, this patented method has many processes and complicated steps, with non-reusable raw materials and high costs in the recycling process.

Additionally, a low-temperature freeze-drying method for preparing silk fibroin powders with a molecular weight below 80 kDa has been proposed. However, due to the small molecular weight of powders prepared using this method, the resulting silk fiborin shows poor mechanical properties and does not bear the characteristics of high-strength biomaterial. It is generally believed that a higher molecular weight (> 100 kDa) and a narrower molecular weight distribution lead to better mechanical properties of the silk fiboroin material. However, the extraction process for macromolecular silk fibroin is very complex. In order to maintain the molecular weight and the conformation of the protein molecular chain, it is necessary to use a suitable dissolving system (e.g., lithium bromide-water binary system etc.), followed by the removal of ions by a suitable method. At present, the commonly used method on the market is dialysis, but dialysis is time-consuming, costly, not suitable for mass production, and difficult to be used for separating silk fiboroin molecules by their molecular weights. In another aspect, the conformations of the silk fibroin molecular chain include random coil, α-helix, and β-sheet, which can transform into each other under certain conditions. Under shear stress, the silk fibroin molecular chain gradually changes from a helix or random coil conformation to a predominantly β-sheet conformation, which may result in protein denaturation or precipitation. The larger the molecular weight of the silk fibroin in solution, the more sensitive it is to shear stress. For this reason, the use of tangential flow in the desalting and concentration of silk fibroin is hardly considered in the prior art.

### SUMMARY

The object of the present invention is to provide a more simplified method for preparing high-concentration, high-molecular-weight silk fibroin aqueous solution, concrete schemes are as follows:
S1: obtaining an inorganic salt solution of silk fibroin;
S2: subjecting the inorganic salt solution of silk fibroin to a membrane clarification process;
S3: performing desalination treatment on the inorganic salt solution of silk fibroin: filtering a clarified solution after the membrane clarification processing through a tangential flow filtration system;
S4: concentrating the silk fibroin solution to obtain an aqueous solution of silk fibroin with a high molecular weight.

In one embodiment of the present invention, in step S1, the inorganic salt solution of silk fibroin refers to an inorganic salt solution of silk fibroin with a number-average molecular weight above 80 kDa.

Preferably, the inorganic salt solution of silk fibroin refers to an inorganic salt solution of silk fibroin with a number-average molecular weight of 80-200 kDa.

Preferably, the number-average molecular weight of the inorganic salt solution of silk fibroin in step S1 is measured by a rheological method.

In one embodiment of the present invention, in step S1, the inorganic salt solution of silk fibroin has a concentration of 0.1%-50% (wt%), preferably 1%-40% (wt%), more preferably 2%-30% (wt%), further preferably 3%-20% (wt%), and furthermore preferably 4%-10% (wt%).

In one embodiment of the present invention, in step S1, the inorganic salt is selected from lithium bromide, sodium thiocyanate, lithium thiocyanate, etc.

In one embodiment of the present invention, in step S1, a method for obtaining the inorganic salt solution of silk fibroin comprises the following steps:
S11: degumming, washing, and drying steps: mixing silkworm cocoons with carbonate and water, and heating the mixture to obtain a degummed silk; then washing and drying the degummed silk;
S12: dissolution step: dissolving the dried degummed silk after step S11 in an inorganic salt solution, and heating the solution to obtain an inorganic salt solution of silk fibroin containing a high concentration of salt.

In one embodiment of the present invention, in step S2, during the membrane clarification process, the membrane material used is a filtration material such as a multilayer glass fiber material, a multilayer filament polypropylene material, a functionalized polyethersulfone material, or a functionalized regenerated cellulose material. The membrane material is preferably a material with properties such as strong clarification ability, high dirt-holding capacity, relatively high total flux, relatively wide chemical compatibility, and relatively low nonspecific adsorption, and multi-layer glass fiber materials are most preferred..

In one embodiment of the present invention, in step S2, process conditions for the membrane clarification processing are as follows: pumping the solution into a filter for filtration which has a filter area of greater than 10 cm², and a pore size of 0.1-50 µm, preferably 0.2-40 µm, 0.5-30 µm, or 1-10 µm, more preferably 1-5 µm, and further preferably 1-2 µm.

In one embodiment of the present invention, in step S3, the desalination processing comprises filtering through an ultrafiltration system which is a tangential flow filtration system. The tangential flow filtration system comprises a membrane module as a filtration component, and the membrane module is comprised of a filtration material such as polyethersulfone, regenerated cellulose, or polyacrylonitrile; preferably, a material with characteristics such as high flow rate and high flux, natural hydrophilicity, and low adsorption is preferable; and a regenerated cellulose material is most preferably.

In one embodiment of the present invention, in step S3, when filtering the clarified solution obtained after the membrane clarification process through the tangential flow filtration system, the tangential flow rate is 0-60 L/min/m², preferably 1-50 L/min/m², 2-40 L/min/m², 3-30 L/min/m², or 4-20 L/min/m², and more preferably 5-10 L/min/m²; the transmembrane pressure is 0.001-3.0 bar, preferably 0.01-2.5 bar or 0.1-2.0 bar, and more preferably 0.15-1.5 bar, such as 0.2-1.0 bar; in the ultrafiltration process, purified water is added to gradually replace the solvent in the silk fibroin solution, with the volume of the purified water added being 1-10 times the volume of the original salt solution, preferably 2-8 times the volume of the original salt solution, more preferably 3-7 times the volume of the original salt solution, or 4-6 times the volume of the original salt solution, and further preferably 5 times the volume of the original salt solution.

In one embodiment of the present invention, in step S4, the obtained aqueous solution of silk fibroin with a high molecular weight refers to an aqueous solution of silk fibroin with a number-average molecular weight above 80 kDa, preferably with a number-average molecular weight of 80-200 kDa.

Preferably, the number-average molecular weight of the aqueous solution of silk fibroin in step S4 is measured by a rheological method.

In one embodiment of the present invention, in step S4, the obtained aqueous solution of silk fibroin with a high molecular weight has a mass fraction of 1 %-40% (wt%), preferably 2%-30% (wt%), more preferably 3%-20% (wt%), and further preferably 4%-10% (wt%), wherein the mass fraction is the ratio of the mass of silk fibroin to the mass of the aqueous solution of silk fibroin.

In one embodiment of the present invention, in step S4, after concentration, the obtained aqueous solution of silk fibroin is poured into a container and stored under refrigeration. The concentration of the aqueous solution of silk fibroin obtained after concentration can be calibrated by a gravimetric method.

In one embodiment of the present invention, in steps S2, S3, and S4, the solution is fed into the filtration system using a pump selected from a diaphragm pump, peristaltic pump, or sanitary lobe pump.

In one embodiment of the present invention, by setting different cut-off pore sizes for the membrane module of the tangential flow filtration system, the aqueous solutions of silk fibroin with different number-average molecular weights are obtained, thereby achieving the fractionation of the aqueous solution of silk fibroin.

The technical effects of the present invention are as follows:
(1) In the present invention, the tangential flow technology is innovatively used to achieve desalination and concentration of the silk fibroin salt solution, so as to directly obtain an aqueous solution of silk fibroin with a number-average molecular weight of 80-200 kDa, which results in unexpected technical effects.
   Unlike conventional vertical filtration, in the tangential flow filtration process, the liquid flows tangentially across the membrane surface, part of the solution is pressed by the transmembrane pressure generated by the fluid to pass through the filter membrane, and the trapped part circulates back through the system. Throughout the whole process, the liquid flows at a certain rate across the surface of the filter membrane and thus continuously scours the surface of the filter membrane, thereby preventing the formation of a gel layer on the surface of the filter membrane. However, in order to fully utilize the advantages of the tangential flow filtration for preparing an aqueous solution of silk fibroin with a number-average molecular weight of 80-200kDa, numerous technical difficulties must be overcome. In the innovation process of present invention, many technical problems such as reducing the shear force of the whole system, avoiding the blockage of the membrane module, and increasing the yield are solved, which greatly improves production efficiency and reduces production costs, provides more opportunities for the production of silk fibroin materials, and enables the present invention to be suitable for the large-scale production of silk fibroin raw materials in industry.
(2) The key to ultrafiltration lies in the selection of process conditions based on a special processing object, e.g., including the selection of a membrane material and transmembrane pressure. In the present invention, by testing different types and specifications of membrane, flow rate and solution concentration, the optimal experimental conditions are determined. Furthermore, the clarification process is generally considered only for products obtained through fermentation to remove impurities such as bacterial fragments from the fermentation solution, and not for degummed silk fibroin. Through long-term exploration and practice, the present invention develops a suitable clarification method that can solve the problem of a part of colloidal particles not dissolved in the lithium bromide solution being deposited on the surface of the membrane and causing the blockage of the membrane pores. This method can not only ensure the yield but also remove the colloidal particles.
(3) In another aspect, the inorganic salt used in the dissolution process of silk fibroin can be directly recycled. In the desalination process of the method of the present invention, the inorganic salt solution (e.g., LiBr) is exchanged with 1-10 volumes of purified water, so that the LiBr solution is subjected to a 1-10-fold dilution. The diluted LiBr solution can be prepared into a solution for dissolving the silk fibroin by directly adding LiBr or recycled to obtain pure lithium bromide. In contrast to conventional methods, a large amount of purified water is used for dialysis, for example, 48 L of purified water is required for dialysis of 200 mL of a 9.3 M LiBr solution of silk fibroin, which makes it challenging to recycle LiBr from such a large volume of solution. Since LiBr is relatively expensive , by utilizing the method of the present invention, costs can be reduced significantly and water resources can be conserved.
(4) Conventional dialysis methods require a total of 4-5 days to treat 1 L of solution, while the method of the present invention requires only 1-2 hours to treat 1 L of solution. By the method of the present invention, an aqueous solution of silk fibroin with a high molecular weight can be obtained at a lower cost and in a shorter time, which provides possibilities for the large-scale preparation of new functional silk fibroin-based materials, thereby further opening up a new prospect for the industrialization of silk fibroins.

### BRIEF DESCRIPTION OF THE DRAWINGS

FIG. 1 shows the membrane microstructure of multilayer glass fiber (left) and polypropylene fiber (right) under an electron microscope.
FIG. 2 shows the membrane surface structure of regenerated cellulose under an electron microscope (top) and the schematic diagram of chemical modification (bottom).
FIG. 3 shows the schematic diagram of a membrane clarification system according to the present invention.
FIG. 4 shows the schematic diagram of a tangential flow filtration system according to the present invention.
FIG. 5 shows the results of a clarification filtration experiments.
FIG. 6 shows the results of multivariate data analysis.
FIG. 7 shows the process curve for buffer exchange of the sample.
FIG. 8 shows the process curve for sample concentration.
FIG. 9 shows the change in turbidity of sample after different process steps.
FIG. 10-1 shows experimental process parameters under a condition of a polypropylene filter/0.45 µm.
FIG. 10-2 shows experimental process parameters under a condition of a polypropylene filter/0.65 µm.
FIG. 10-3 shows experimental process parameters under a condition of a glass fiber filter/0.65 µm;
FIG. 10-4 shows experimental process parameters under a condition of a polypropylene filter/1.2 µm.
FIG. 10-5 shows experimental process parameters under a condition of a glass fiber filter/1.2 µm;
FIG. 10-6 shows experimental process parameters under a condition of a polypropylene filter/3 µm.
FIG. 10-7 shows experimental process parameters under a condition of a polypropylene filter/5 µm.

### DETAILED DESCRIPTIONS

The present invention will be further illustrated with reference to the following specific examples. It should be understood that these examples are merely intended to illustrate the present invention rather than limit the scope of protection of the present invention. In addition, it should be understood that various changes or modifications may be made by those skilled in the art after reading the instructions of the present invention, and these equivalents also fall within the scope of protection of the present invention.

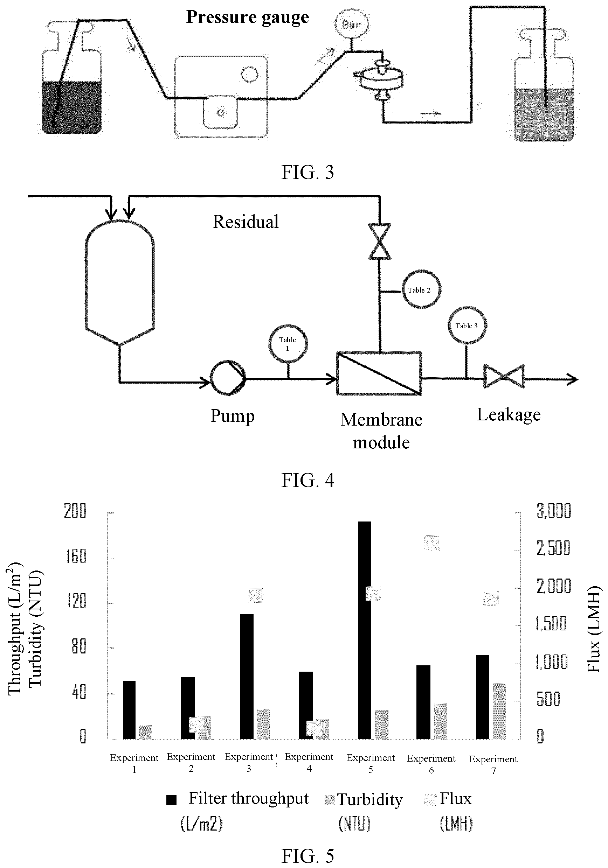

The method for preparing a controllable aqueous solution of high-concentration silk fibroin by tangential flow ultrafiltration technology of the present invention comprises the following steps:
Step S1: obtaining an inorganic salt solution of silk fibroin, with specific procedures as follows:
   S11: degumming, washing, and drying steps: mixing silkworm cocoon with carbonate (e.g., sodium carbonate or sodium bicarbonate) and water, and heating the mixture to obtain a degummed silk; and washing and drying the degummed silk;
   S12: dissolution step: dissolving the dried degummed silk after step S11 in an inorganic salt solution, and heating the solution to obtain an inorganic salt solution of silk fibroin containing a high concentration of salt;
   wherein, the inorganic salt solution of silk fibroin has a concentration of 0.1 wt%-50 wt%, preferably 4 wt%-10 wt%, and the inorganic salt used is selected from lithium bromide, sodium thiocyanate, lithium thiocyanate, etc.
Step S2: subjecting the inorganic salt solution of silk fibroin to membrane clarification process, with specific procedures as follows:
   a. determining the initial turbidity of a sample stock;
   b. assembling a filtration device according to a schematic diagram, as shown in FIG. 3;
   c. rinsing and soaking a test pipeline with a 1 M NaOH solution, followed by rinsing with purified water;
   d. inserting a sample inlet tube into a sample flask, adjusting the pump speed to an appropriate rate, starting filtration, and recording filtration time, filtrate volume, pressure gauge reading, etc., in real-time;
   e. when a feed liquid in the sample flask is filtered out or the pressure rises to 1.5 bar, stopping the filtration test;
   f. collecting the filtrate and determining the turbidity of the filtered sample.

During the membrane clarification process, the membrane material used is a filtration material such as a multilayer glass fiber material, a multilayer filament polypropylene material, a functionalized polyethersulfone material, or a functionalized regenerated cellulose material; and a material with characteristics such as high flow rate and high flux, natural hydrophilicity, and low adsorption is preferable.

In the study of the present invention, a laboratory small-scale filterability test is performed on the dissolved sample feed liquid by using clarification filters of two different membrane materials, namely, a glass fiber material and a polypropylene material, so as to evaluate important properties the of filters such as the filtration throughput, filtration rate of each of the filters, and the turbidity of the filtered sample, thereby providing a necessary basis for selecting clarification filters for large-scale production in the future.

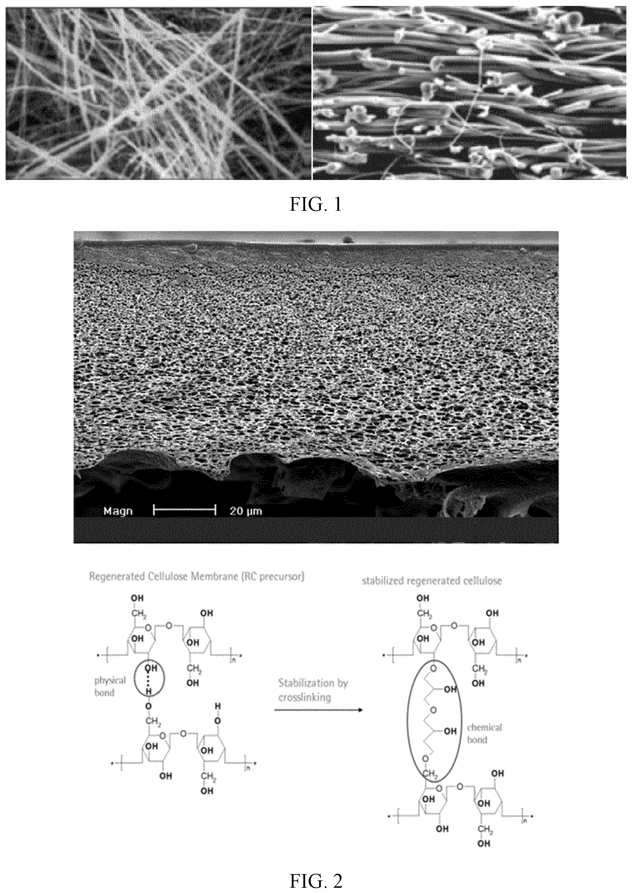

In one embodiment of the present invention, the clarification filter used is a deep prefilter of a multilayer glass fiber material, with two nominal pore sizes of 0.65 µm and 1.2 µm, which has high dirt-holding capacity, and is particularly suitable for the filtration of liquids with high colloid and particle content, and is widely used in the pre-filtration of samples with high viscosity and samples with high colloid and lipid content. Its membrane microstructure is shown in FIG. 1 (left).

In one embodiment of the present invention, the clarification filter used is a deep prefilter of a multilayer filament polypropylene material, with a nominal pore size in a range of 0.45-50 µm, which has characteristics of relatively strong clarification ability, relatively high total flux, relatively wide chemical compatibility, and relatively low nonspecific adsorption, and is often used for vaccines and blood products. Its membrane microstructure is shown in FIG. 1 (right).

According to the properties of the silk fibroin solution, in the selection of process conditions for the membrane clarification process in the present invention, two membrane materials (glass fiber material and polypropylene material) and deep prefilters with five pore sizes are selected for clarification experiments. The glass fiber material has two pore sizes of 0.65 µm and 1.2 µm, and a total of 7 experiments were conducted. The experimental design is shown in Table 1.

**Table 1. Clarification experiments of silk fibroin**

| Serial number | Name of experiment | Membrane material | Pore size |
|---|---|---|---|
| 1 | Experiment 1 | Polypropylene | 0.45 µm |
| 2 | Experiment 2 | Polypropylene | 0.65 µm |
| 3 | Experiment 3 | Glass fiber | 0.65 µm |
| 4 | Experiment 4 | Polypropylene | 1.2 µm |
| 5 | Experiment 5 | Glass fiber | 1.2 µm |
| 6 | Experiment 6 | Polypropylene | 3.0 µm |
| 7 | Experiment 7 | Polypropylene | 5.0 µm |

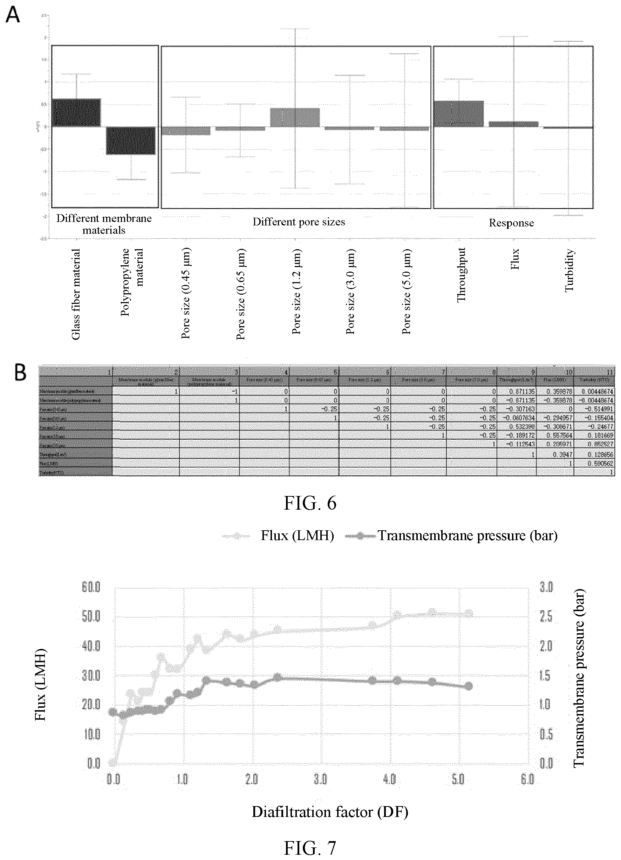

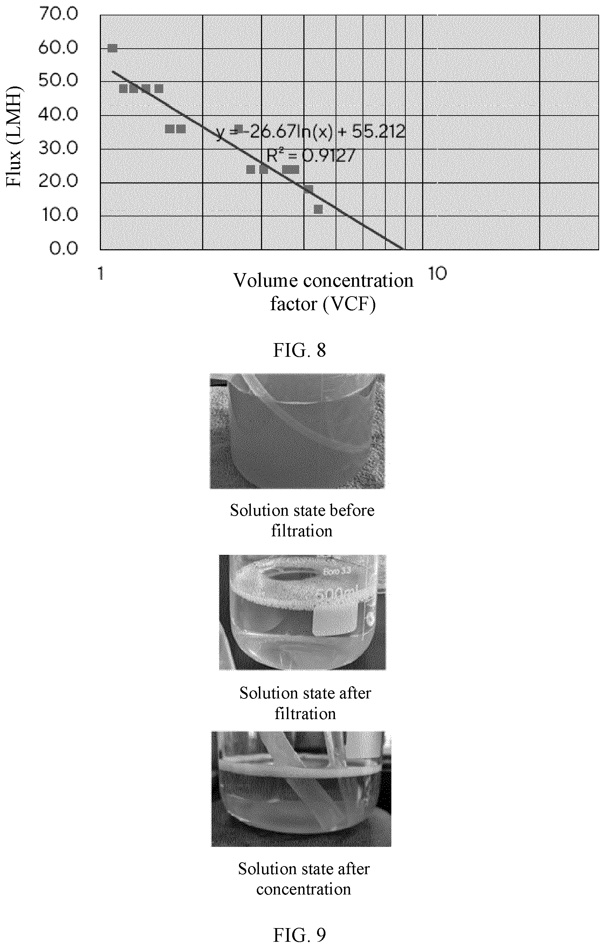

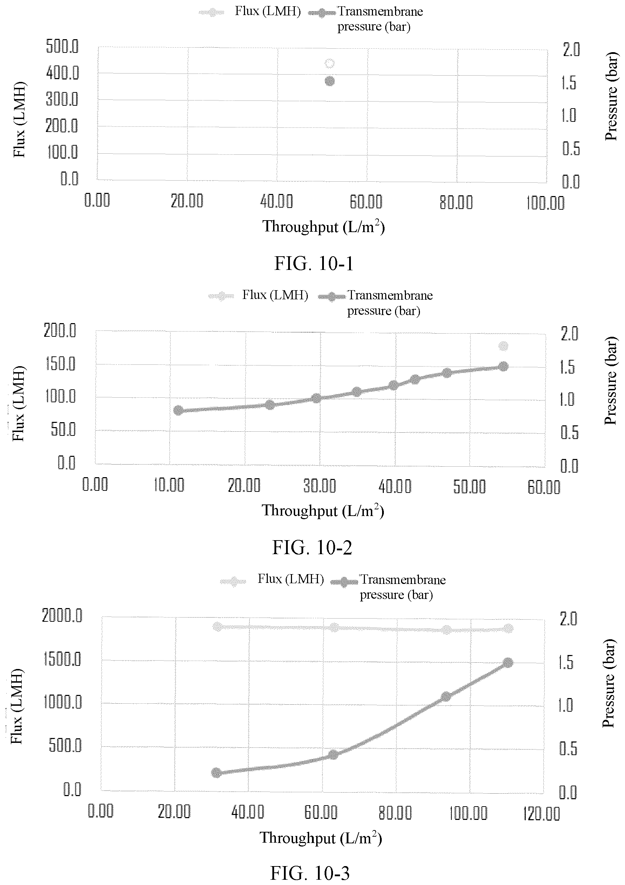

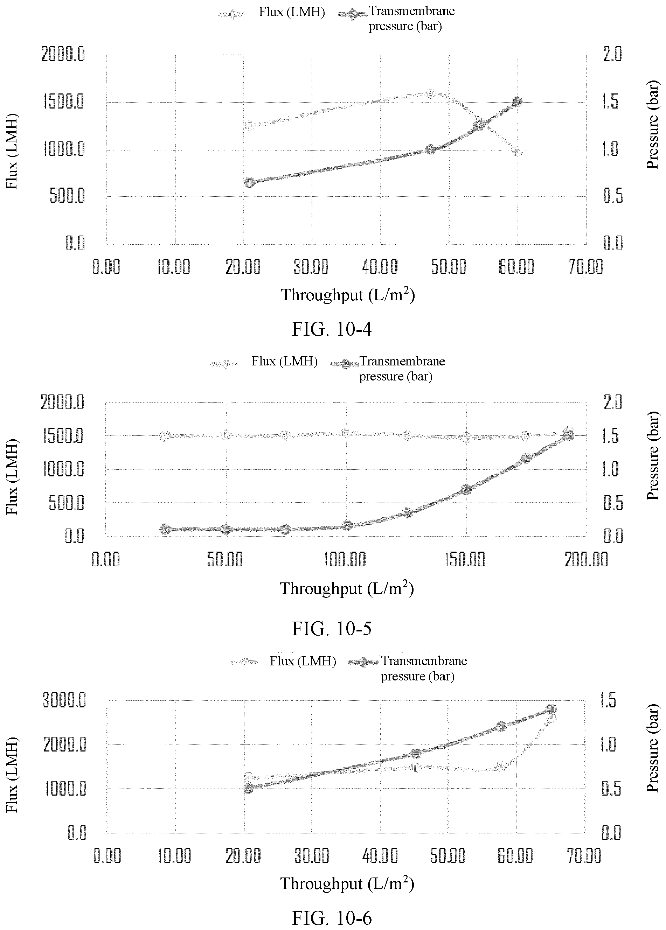

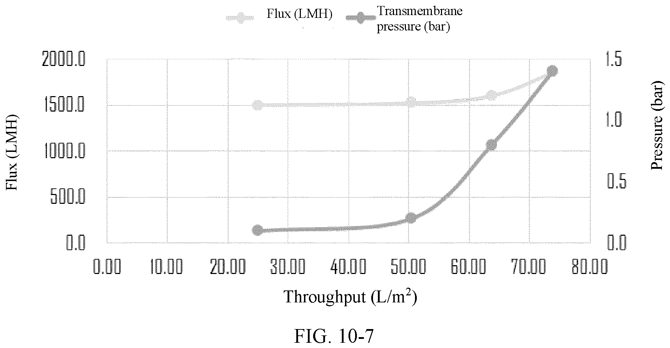

The time, system pressure, and filtration volume during the clarification is recorded, and the throughput and flux of different filters on the dissolved silk fibroin samples were calculated. The filtered samples were well mixed, and the turbidity of each sample was determined. The experimental results are shown in FIG. 5 (see FIG. 10 for the original experimental data). After processing with different filters, the turbidity of the samples decreased from 244 NTU at the beginning to less than 40 NTU. A visual comparison of the samples before and after filtration (as shown in FIG. 9) showed that the clarity of the samples after filtration was significantly improved. This indicates that the clarification process can be effective in removing insoluble impurities such as colloidal particles from the dissolved sample. The analysis of the above results by using SIMCA multivariable data analysis software showed that in terms of different membrane materials and pore sizes, the membrane materials were significantly correlated with the filter throughput (shown in the leftmost column of FIG. 6A), among which the glass fiber material showed a positive correlation with a correlation coefficient of 0.87 (shown in the leftmost column of FIG. 6A). This indicates that the glass fiber filter can significantly increase the sample processing capacity per unit area of filter membrane and reduce the production cost. There is no obvious correlation between pore size and filter throughput. In addition, the membrane material and pore size have little effect on the flux of the filters and the turbidity of the filtered samples. Subsequently, the glass fiber membrane material can be used to derive the optimal flux by process optimization.

Step S3: performing desalination process on the inorganic salt solution of silk fibroin: filtering a clarified solution after the membrane clarification process through a tangential flow filtration system, with specific procedures as follows:
a. installing a tangential flow filtration system as shown in FIG. 4;
b. rinsing a pipeline of the system with a 1 M NaOH solution and purified water;
c. determining water flux of a membrane module: reading the volume of purified water collected at the permeate end within a certain period of time;
d. equilibrating the membrane module: inserting a sample inlet tube in a sample flask containing LiBr solution or purified water for 5 min of circulation, so as to equilibrate the membrane module;
e. tangential flow experiment: adjusting the tangential flow rate to 0-60 L/min/m², preferably 5.0 L/min/m²; adjusting the transmembrane pressure to 0.001-3.0 bar, preferably 0.15-1.5 bar; performing an ultrafiltration experiment under a condition that the volume of a liquid for buffer exchange was 0-10 times the volume of the original inorganic salt solution to exchange the target protein into purified water;
f. rinsing the pipeline of the system with a 1 M NaOH solution and purified water;
g. determining water flux of the membrane module: reading the volume of purified water collected at the permeate end within a certain period of time;
h. the solid content for silk fibroin concentration is calibrated by a gravimetric method. The membrane module of the tangential flow filtration system uses a filtration material such as polysulfone, regenerated cellulose, or polyacrylonitrile, preferably a material with characteristics such as high flow rate and high flux, natural hydrophilicity, and low adsorption.

In one embodiment of the present invention, the tangential flow filtration system has a structure as follows: the membrane module is fixed in the system by a clamp, with sample inlet and outlet tubes connected to both ends for convenience of use. An ultrafiltration membrane module of a stabilized regenerated cellulose material is used. The material itself has natural hydrophilicity and low adsorption, and thus has high flow rate and high flux. The membrane surface was specially modified and chemically treated, which provides the material with better chemical compatibility and membrane regeneration function than that of conventional polysulfone and general regenerated cellulose materials. FIG. 2 shows the membrane surface structure under an electron microscope and chemical modification mechanism.

In one embodiment of the present invention, the clarified solutions obtained after the membrane clarification process are mixed as a starting material and subjected to be replaced with 5 volumes of a liquid to exchange the sample into the target purified water buffer. Curves of flow rate of permeate side and transmembrane pressure (TMP) vs. the liquid exchange volume during the experiment are shown in FIG. 7. It can be seen from the tangential flow filtration curve that the flow rate of permeate side is relatively low at the initial stage of solvent exchange due to the high viscosity of the starting sample material. As the washing and filtration volumes increases, the LiBr concentration in the sample gradually decreases, and the viscosity of the sample also decreases, leading to an increase in the flow rate of permeate side. When the LiBr content in the sample is reduced to a relatively low level, the transmembrane pressure of the system gradually decreases. The experiment lasts for 53 min, and the flow rate of permeate side does not show significant attenuation, indicating that the sample is stable during the buffer exchange process, and that the process parameters used are reasonable, thus providing reference for the subsequent large-scale processes. The protein content of the sample after diafiltration is 3.4%.

Based on the above experimental results, silk fibroin in the aqueous phase is concentrated, and the change in flow rate of permeate side with increasing concentration factor is investigated. The experimental results are shown in FIG. 8: the solution is concentrated from 640 mL to 145 mL, with a volume concentration factor of 4.41. The concentrated sample is shown in FIG. 9, which was still in a clarified state. The protein content of the sample after concentration was 12.4%. This suggests that silk fibroin in the aqueous phase is stable under the above-mentioned concentration process conditions. The product yield is 82.63%.

Step S4: concentrating the silk fibroin solution to obtain an aqueous solution of silk fibroin with a high molecular weight, with specific procedures as follows: the method and process are the same as those of step S3, except that the purified water inlet port in step e was closed. In step S4, the obtained aqueous solution of silk fibroin with a high molecular weight has a number-average molecular weight of 80-200 kDa (as measured by a rheologic method) and a mass fraction of 1%-40%.

After concentration, the obtained silk fibroin solution is poured into a container and stored under refrigeration.

The present invention will be further illustrated with reference to the drawings and the specific examples below. In the following examples, the method and procedures are basically the same, but the parameters and settings may vary.

### Example 1

S1: Preparation of salt solution of silk fibroin: Silk was degummed, washed with water, and dried. The obtained silk fibroin was dissolved in a 9.3 M lithium bromide (LiBr) solution to form a salt solution with a concentration of 10 g/100 mL.
S2: Clarification experiment:
   a. The initial turbidity of an original sample solution was determined to be 244 NTU.
   b. A filtration device was assembled according to a schematic diagram, and a 0.45 µm polypropylene membrane filter was selected, as shown in FIG. 3.
   c. A test pipeline was rinsed with and soaked in a 1 M NaOH solution and then rinsed with purified water.
   d. The inlet tube was inserted into a sample flask, the pump speed was adjusted to about 20-30 mL/min, and the filtration was started. The filtration time, filtrate volume, pressure gauge reading, etc. were recorded in real-time, as shown in FIG. 5.
   e. When the feed liquid in the sample flask was filtered out, the filtration test was stopped.
   f. The filtrate was collected, and the turbidity of the filtered sample was determined to be 10 NTU.
S3: Desalination of the salt solution of silk fibroin:
   a. A tangential flow filtration system was assembled and a membrane module with a 50 kD cutoff pore size was selected, as shown in FIG. 4.
   b. The pipeline of the system was rinsed with a 1 MNaOH solution and purified water.
   c. Equilibration of a membrane module: The sample inlet tube was inserted into a sample flask containing LiBr solution or purified water for 5 min of circulation, so as to equilibrate the membrane module.
   d. Tangential flow experiment: The tangential flow rate was adjusted to 5.0 L/min/m² and the clarified samples were mixed as a starting material and subjected to be replaced with 5 volumes of a liquid to exchange the sample into the target purified water buffer.
   e. The pipeline of the system was rinsed with a 1 MNaOH solution and purified water.
   f. The solid content for silk fibroin concentration was calibrated as 3.4% by a gravimetric method.
   g. The number-average molecular weight of silk fibroin was measured as 85 kD by a rheological method.
S4: Concentration of the silk fibroin solution:
   The aqueous solution of silk fibroin obtained in step S3 was concentrated using the same method as in step S3, except that the purified water inlet port in step d was closed. The solid content for silk fibroin concentration was calibrated as 12.4% by a gravimetric method.

### Example 2

S1: Preparation of salt solution of silk fibroin: Silk was degummed, washed with water, and dried. The obtained silk fibroin was dissolved in a 9.3 M lithium bromide (LiBr) solution to give a salt solution with a concentration of 10 g/100 mL.
S2: Clarification experiment:
   a. The filtration device was assembled according to the schematic diagram, and a 0.45 µm polypropylene membrane filter was selected, as shown in FIG. 3.
   b. A test pipeline was rinsed with and soaked in a 1 M NaOH solution and then rinsed with purified water.
   c. The inlet tube was inserted into a sample flask, the pump speed was adjusted to about 20-30 mL/min, and the filtration was started.
   d. When the feed liquid in the sample flask was filtered out, the filtration test was stopped.
   e. The filtrate was collected.
S3: Desalination of the salt solution of silk fibroin:
   a. A tangential flow filtration system was assembled and a membrane module with a 100 kD cutoff pore size was selected, as shown in FIG. 4.
   b. The pipeline of the system was rinsed with a 1 MNaOH solution and purified water.
   c. Equilibration of a membrane module: The sample inlet tube was inserted into a sample flask containing LiBr solution or purified water for 5 min of circulation, so as to equilibrate the membrane module.
   d. Tangential flow experiment: The tangential flow rate was adjusted to 1.0 L/min/m² and the clarified samples were mixed as a starting material and subjected to be replaced with 5 volumes of a liquid to exchange the sample into the target purified water buffer.
   e. The pipeline of the system was rinsed with a 1 MNaOH solution and purified water.
   f. The solid content for silk fibroin concentration was calibrated as 3.2% by a gravimetric method.
   g. The number-average molecular weight of silk fibroin was measured as 124 kD by a rheological method.
S4: Concentration of the silk fibroin solution:
   The aqueous solution of silk fibroin obtained in step (3) was concentrated using the same method as in step (3), except that the purified water inlet port in step d was closed. The solid content for silk fibroin concentration was calibrated as 11.5% by a gravimetric method.

### Example 3

S1: Preparation of salt solution of silk fibroin: Silk was degummed, washed with water, and dried. The obtained silk fibroin was dissolved in a 9.3 M lithium bromide (LiBr) solution to give a salt solution with a concentration of 5g/100 mL.
S2: Clarification experiment:
   a. The filtration device was assembled according to the schematic diagram, and a 0.45 µm polypropylene membrane filter was selected, as shown in FIG. 3.
   b. A test pipeline was rinsed with and soaked in a 1 M NaOH solution and then rinsed with purified water.
   c. The inlet tube was inserted into a sample flask, the pump speed was adjusted to about 20-30 mL/min, and the filtration was started.
   d. When the feed liquid in the sample flask was filtered out, the filtration test was stopped.
   e. The filtrate was collected.
S3: Desalination of the salt solution of silk fibroin:
   a. A tangential flow filtration system was assembled and a membrane module with a 150 kD cutoff pore size was selected, as shown in FIG. 4.
   b. The pipeline of the system was rinsed with a 1 MNaOH solution and purified water.
   c. Equilibration of a membrane module: The sample inlet tube was inserted into a sample flask containing LiBr solution or purified water for 5 min of circulation, so as to equilibrate the membrane module.
   d. Tangential flow experiment: The tangential flow rate was adjusted to 3.0 L/min/m² and the clarified samples were mixed as a starting material and subjected to be replaced with 5 volumes of a liquid to exchange the sample into the target purified water buffer.
   e. The pipeline of the system was rinsed with a 1 MNaOH solution and purified water.
   f. The solid content for silk fibroin concentration was calibrated as 2.7% by a gravimetric method.
   g. The number-average molecular weight of silk fibroin was measured as 169 kD by a rheological method.
S4: Concentration of the silk fibroin solution:
   The aqueous solution of silk fibroin obtained in step S3 was concentrated using the same method as in step S3, except that the purified water inlet port in step (d) was closed. The solid content for silk fibroin concentration was calibrated as 9.4% by a gravimetric method.

### Example 4

Provided is a method for preparing a controllable high-concentration silk fibroin solution by tangential flow ultrafiltration technology, comprising the following steps:
S1: An inorganic salt solution of silk fibroin was acquired by degumming, washing and drying: Silkworm cocoons were mixed with an inorganic salt (sodium carbonate) and water then heated to give a degummed silk. The degummed silk was washed and dried. The dried and degummed silk was dissolved in an inorganic salt solution and heated to give a salt solution of silk fibroin with a number-average molecular weight of 80 kDa-200 kDa and a concentration of 5 wt%.
S2: The inorganic salt solution of silk fibroin was subjected to membrane clarification process. A multilayer glass fiber membrane was used. The process condition for the membrane clarification processing is as follows: the solution was pumped into the filter with a filtration area of greater than 10 cm² and a pore size of 1.2 µm.
S3: Desalination of the inorganic salt solution of silk fibroin: The clarified solution obtained from the membrane clarification process was filtered through a tangential flow filtration system, and functionalized regenerated cellulose was selected as the material for the membrane module. When the clarified solution obtained from the membrane clarification processing was filtered through a tangential flow filtration system for ultrafiltration, the cutoff pore size was 100 kDa, the tangential flow rate is 5.0 L/min/m², the transmembrane pressure was 1 bar, and the solution was fed into the filtration system using a pump.
S4: The silk fibroin solution was concentrated to give an aqueous solution of silk fibroin with a number-average molecular weight of 80 kDa-200 kDa and a mass fraction of 8.6%. The solution was fed into the filtration system using a pump.

The pump used in steps S2, S3, and S4 was a peristaltic pump.

### Example 5

Provided is a method for preparing a controllable high-concentration silk fibroin solution by tangential flow ultrafiltration technology, comprising the following steps:
S1: An inorganic salt solution of silk fibroin was acquired by degumming, washing and drying: Silkworm cocoons were mixed with an inorganic salt (sodium bicarbonate) and water then heated to give a degummed silk. The degummed silk was washed and dried. The dried and degummed silk was dissolved in an inorganic salt solution and heated to give a salt solution of silk fibroin with a number-average molecular weight of 80 kDa-200 kDa and a concentration of 8 wt%.
S2: The inorganic salt solution of silk fibroin was subjected to membrane clarification process. The filtration material of the membrane module was selected from a multilayer glass fiber, a multilayer filament polypropylene, functionalized polyethersulfone, functionalized regenerated cellulose, etc. The process condition for the membrane clarification processing is as follows: the solution was pumped into the filter with a filtration area of greater than 10 cm² and a pore size of 10 µm.
S3: Desalination of the inorganic salt solution of silk fibroin: The clarified solution obtained from the membrane clarification process was filtered through a tangential flow filtration system, and The material for the membrane module was selected from polyethersulfone, regenerated cellulose, or functionalized regenerated cellulose. When the clarified solution obtained from the membrane clarification process was filtered through a tangential flow filtration system for ultrafiltration, the cutoff pore size was 100 kDa, the tangential flow rate is 10 L/min/m², the transmembrane pressure was 0.15 bar, and the solution was fed into the filtration system using a pump.
S4: The silk fibroin solution was concentrated to give an aqueous solution of silk fibroin with a number-average molecular weight of 80 kDa-200 kDa and a mass fraction of 10.7%. The solution was fed into the filtration system using a pump. The pump used in steps S2, S3, and S4 was a sanitary lobe pump.

### Example 6

Provided is a method for preparing a controllable high-concentration silk fibroin solution by tangential flow ultrafiltration technology, comprising the following steps:
S1: An inorganic salt solution of silk fibroin was acquired by degumming, washing and drying: Silkworm cocoons were mixed with an inorganic salt (sodium carbonate) and water then heated to give a degummed silk. The degummed silk was washed and dried. The dried and degummed silk was dissolved in an inorganic salt solution and heated to give a salt solution of silk fibroin with a number-average molecular weight of 80 kDa-200 kDa and a concentration of 8 wt%.
S2: The inorganic salt solution of silk fibroin was subjected to membrane clarification process. The membrane module was of a functionalized polyethersulfone material. The process condition for the membrane clarification processing is as follows: the solution was pumped into the filter with a filtration area of greater than 10 cm² and a pore size of 20 µm.
S3: Desalination of the inorganic salt solution of silk fibroin: The clarified solution obtained from the membrane clarification process was filtered through a tangential flow filtration system, and the material for the membrane module was selected from polyethersulfone, regenerated cellulose, or polyacrylonitrile. When the clarified solution obtained from the membrane clarification process was filtered through a tangential flow filtration system for ultrafiltration, the cutoff pore size was 100 kDa, the tangential flow rate is 10 L/min/m², the transmembrane pressure was 1.5 bar, and the solution was fed into the filtration system using a pump.
S4: The silk fibroin solution was concentrated to give an aqueous solution of silk fibroin with a number-average molecular weight of 80 kDa-200 kDa and a mass fraction of 11.4%. The solution was fed into the filtration system using a pump.

The pump used in steps S2, S3, and S4 was a diaphragm pump.

### Comparative Example

A conventional method for preparing high-concentration silk fibroin solutions was used, and the procedures are as follows:
NaHCO₃ degummed silk (including discarded silkworm silk and cocoon silk) was used to prepare the original silk solution. Specifically, 10 g of discarded silkworm silk or cocoon silk was degummed in 2000 mL of 0.5 wt% aqueous NaHCO₃ solution (bath ratio: 1: 200 g/mL) at 100°C for 30 min, followed by degumming for another 30min under the same conditions in fresh NaHCOs solution.After degumming, the silk was washed three times with hot water and then rinsed with deionized water. The degummed silk was then dried in an oven at 65 °C for 24 h, followed by laying on a tray and drying again in an oven at 45 °C for 24 h. The dried fibers were stored in a vacuum desiccator. Approximately 25% weight loss was observed after drying.

Dissolution: The degummed silk was dissolved in a 9.3 mol/L aqueous LiBr solution (bath ratio: 1:10 g/mL) at 60 °C with stirring until the silk was completely dissolved to give a light yellow-brown opaque viscous solution.

Diafiltration: After coarsely filtering the foam and impurities with multiple layers of gauze, the silk fibroin solution was placed in a diafiltration bag with a cutoff molecular weight of 14000 Da and diafiltrated with deionized water for 3 days with the water being refreshed every three hours. The diafiltrated silk fibroin solution was centrifuged at 6000 r/min for 6 min to remove a small amount of precipitate, and the supernatant was stored in a refrigerator at 4 °C for later use. The concentration of the silk fibroin solution prepared by this method was calibrated as about 4 wt% by a gravimetric method.

Concentration: The dilute solution was placed in a diafiltration bag with a cutoff molecular weight of 14000 Da and concentrated using a 10 wt% PEG solution (2000 mL). The desired high-concentration regenerated silk fibroin solution was obtained by controlling the concentration time. The concentration of the silk fibroin solution prepared by this method was calibrated as about 13 wt%-19 wt% by a gravimetric method. The solution was stored in a refrigerator at 4 °C for later use.

The conventional diafiltration method took 3 days for processing 1 L of solution. Concentrating with PEG solution further prolongs the total processing period to a total of 4-5 days.

## Claims

1. A method for preparing a controllable high-concentration silk fibroin solution by tangential flow ultrafiltration technology, comprising the following steps:
S1: obtaining an inorganic salt solution of silk fibroin;
S2: subjecting the inorganic salt solution of silk fibroin to membrane clarification process;
S3: performing desalination process on the inorganic salt solution of silk fibroin: filtering a clarified solution obtained from the membrane clarification process through a tangential flow filtration system;
S4: concentrating the silk fibroin solution to obtain an aqueous solution of silk fibroin with a high molecular weight.

2. The method for preparing a controllable high-concentration silk fibroin solution using tangential flow ultrafiltration technology according to claim 1,, **characterized in that**, in step S3 filtration in the desalination process is performed through an ultrafiltration system , which is a tangential flow filtration system; the tangential flow filtration system comprises a membrane module as a filtration component, and the membrane module is of a filtration material such as polyethersulfone, regenerated cellulose, or polyacrylonitrile; preferably, a material with characteristics such as high flow rate and high flux, natural hydrophilicity, and low adsorption; and most preferably, a regenerated cellulose material.

3. The method for preparing a controllable high-concentration silk fibroin solution using tangential flow ultrafiltration technology according to claim 2, **characterized in that** in step S3, , when filtering the clarified solution obtained from the membrane clarification process through the tangential flow filtration system, the tangential flow rate is 0-60 L/min/m², preferably 1-50 L/min/m², 2-40 L/min/m², 3-30 L/min/m², or 4-20 L/min/m², and more preferably 5-10 L/min/m²; the transmembrane pressure is 0.001-3.0 bar, preferably 0.01-2.5 bar, 0.1-2.0 bar, and more preferably 0.15-1.5 bar, such as 0.2-1.0 bar; in the ultrafiltration process, purified water is added to gradually replace the solvent in the silk fibroin solution, with the volume of the purified water added being 1-10 times the volume of the original salt solution, such as 2-8 times the volume of the original salt solution, preferably 3-7 times the volume of the original salt solution or 4-6 times the volume of the original salt solution, and more preferably 5 times the volume of the original salt solution.

4. The method for preparing a controllable high-concentration silk fibroin solution using tangential flow ultrafiltration technology according to any one of claims 1-3, **characterized in that**, in step S1 , the inorganic salt solution of silk fibroin refers to an inorganic salt solution of silk fibroin with a number-average molecular weight of 80 kDa or greater, and preferably, the inorganic salt solution of silk fibroin refers to an inorganic salt solution of silk fibroin with a number-average molecular weight of 80 kDa-200 kDa.

5. The method for preparing a controllable high-concentration silk fibroin solution using tangential flow ultrafiltration technology according to any one of claims 1-4, **characterized in that**, in step S1, the inorganic salt solution of silk fibroin has a concentration of 0.1-50 wt%, preferably 1-40 wt%, more preferably 2-30 wt%, even more preferably 3-20 wt%, and most preferably 4-10 wt%.

6. The method for preparing a controllable high-concentration silk fibroin solution using tangential flow ultrafiltration technology according to claim 5, **characterized in that** the inorganic salt is selected from lithium bromide, sodium thiocyanate, lithium thiocyanate, etc.

7. The method for preparing a controllable high-concentration silk fibroin solution using tangential flow ultrafiltration technology according to any one of claims 1-6, **characterized in that**, in step S1, the method for acquiring the inorganic salt solution of silk fibroin comprises the following steps:
S11: degumming, washing, and drying steps: mixing silkworm cocoons with a carbonate and water, and heating the mixture to obtain a degummed silk; and washing and drying the degummed silk;
S12: dissolution step: dissolving the dried and degummed silk obtained from S11 in an inorganic salt solution, and heating to give the inorganic salt solution of silk fibroin with a high salt concentration.

8. The method for preparing a controllable high-concentration silk fibroin solution using tangential flow ultrafiltration technology according to any one of claims 1-7, **characterized in that**, in step S2, during the membrane clarification process, the membrane is of a filtration material such as a multilayer glass fiber, a multilayer filament polypropylene, functionalized polyethersulfone, or functionalized regenerated cellulose, preferably, a material with a strong clarification capacity, high dirt-holding capacity, high total flux, broad chemical compatibility, and low nonspecific adsorption, and a multilayer glass fiber is most preferable.

9. The method for preparing a controllable high-concentration silk fibroin solution using tangential flow ultrafiltration technology according to any one of claims 1-8, **characterized in that**, in step S2, the process conditions for the membrane clarification process comprise: pumping the solution into the filter with a filtration area of greater than 10 cm² and a pore size of 0.1-50 µm, preferably 0.2-40 µm, 0.5-30 µm or 1-10 µm, more preferably 1-5 µm, and even more preferably 1-2 µm.

10. The method for preparing a controllable high-concentration silk fibroin solution using tangential flow ultrafiltration technology according to any one of claims 1-9, **characterized in that**, in step S4, the obtained aqueous solution of silk fibroin with high molecular weight refers to an aqueous solution of silk fibroin with a number-average molecular weight of 80 kDa or greater, and preferably, an aqueous solution of silk fibroin with a number-average molecular weight of 80 kDa-200 kDa.

11. The method for preparing a controllable high-concentration silk fibroin solution using tangential flow ultrafiltration technology according to any one of claims 1-10, **characterized in that** the number-average molecular weights of the inorganic salt solution of silk fibroin in S1 and the aqueous solution of silk fibroin in S4 are measured by a rheological method.

12. The method for preparing a controllable high-concentration silk fibroin solution using tangential flow ultrafiltration technology according to any one of claims 1-11, **characterized in that**, in step S4, the obtained aqueous solution of silk fibroin with high molecular weight has a mass fraction of 1-40 wt%, preferably a mass fraction of 2-30 wt%, more preferably 3-20 wt%, and even more preferably 4%-10 wt%; the mass fraction is the ratio of the mass of silk fibroin to the mass of aqueous solution of silk fibroin.
